# EUROPEAN PATENT SPECIFICATION

(11) **EP 1 421 215 B1**
(45) Date of publication and mention of the grant of the patent: **16.03.2011**
(21) Application number: 02763327.0
(22) Date of filing: 23.07.2002
(51) Int. Cl.: C12Q 1/68

(54) **METHODS FOR EVALUATING PATHOLOGIC CONDITIONS USING EXTRACELLULAR RNA**
VERFAHREN ZUR BEWERTUNG PATHOLOGISCHER KRANKHEITSZUSTÄNDE UNTER VERWENDUNG EXTRAZELLULÄRER RNA
METHODES D'EVALUATION D'AFFECTIONS PATHOLOGIQUES UTILISANT UN ARN EXTRACELLULAIRE

(30) Priority: 25.07.2001 US 308054 P
(43) Date of publication of application: 26.05.2004
(73) Proprietor: Oncomedx Inc., Long Valley, NJ 07853 (US)
(72) Inventor: KOPRESKI, Mike, Long Valley, NJ 07853 (US)
(74) Representative: Grünecker, Kinkeldey, Stockmair & Schwanhäusser Anwaltssozietät
(86) International application number: PCT/US2002/023373
(87) International publication number: WO 2003/009806

(56) References cited:
- WO-A-97/35589
- WO-A-98/14617
- TAKAMIYAGI A ET AL: "Quantitative analysis of ferrochelatase mRNA in blood cells of erythropoietic protoporphyria patients." JOURNAL OF DERMATOLOGICAL SCIENCE. FEB 1996, vol. 11, no. 2, February 1996 (1996-02), pages 154-160, XP002359998 ISSN: 0923-1811
- GARBARZ M ET AL: "SPECTRIN BETA-TANDIL A NOVEL SHORTENED BETA-CHAIN VARIANT ASSOCIATED WITH HEREDITARY ELLIPTOCYTOSIS IS DUE TO A DELETIONAL FRAMESHIFT MUTATION IN THE BETA SPECTRIN GENE" BLOOD, vol. 80, no. 4, 1992, pages 1066-1073, XP002359999 ISSN: 0006-4971
- LASHEEB A S ET AL: "Semen characteristics in HIV-1 positive men and the effect of semen washing." GENITOURINARY MEDICINE. AUG 1997, vol. 73, no. 4, August 1997 (1997-08), pages 303-305, XP009058952 ISSN: 0266-4348
- MERMIN J H ET AL: "Detection of human immunodeficiency virus DNA and RNA in semen by the polymerase chain reaction." THE JOURNAL OF INFECTIOUS DISEASES. OCT 1991, vol. 164, no. 4, October 1991 (1991-10), pages 769-772, XP009058999 ISSN: 0022-1899
- MONTEYNE P ET AL: "Expression of costimulatory molecules and cytokines in CSF and peripheral blood mononuclear cells from multiple sclerosis patients." ACTA NEUROLOGICA BELGICA. MAR 1999, vol. 99, no. 1, March 1999 (1999-03), pages 11-20, XP009058995 ISSN: 0300-9009
- SERRA C ET AL: "Multiple sclerosis and multiple sclerosis-associated retrovirus in Sardinia" NEUROLOGICAL SCIENCES, vol. 22, no. 2, April 2001 (2001-04), pages 171-173, XP002360000 & THIRD ALGHERO INTERNATIONAL CONFERENCE ON MULTIPLE SCLEROSIS: PATHOGENESIS AND THERAPY; ALGHERO, SARDINIA, ITALY; MAY 21-24, 2000 ISSN: 1590-1874
- KOPRESKI M S ET AL: "Cellular- versus extracellular-based assays. Comparing utility in DNA and RNA molecular marker assessment." ANNALS OF THE NEW YORK ACADEMY OF SCIENCES. APR 2000, vol. 906, April 2000 (2000-04), pages 124-128, XP009058975 ISSN: 0077-8923
- MESSNER BARBARA ET AL: "Expression of messenger RNA of the cardiac isoforms of troponin T and I in myopathic skeletal muscle" AMERICAN JOURNAL OF CLINICAL PATHOLOGY, vol. 114, no. 4, October 2000 (2000-10), pages 544-549, XP009058987 ISSN: 0002-9173
- KAMM R C ET AL: "NUCLEIC-ACID CONCENTRATIONS IN NORMAL HUMAN PLASMA" CLINICAL CHEMISTRY, vol. 18, no. 6, 1972, pages 519-522, XP002360001 ISSN: 0009-9147
- SHUTACK J G ET AL: "A study of the RNA levels of normal blood serum." THE JOURNAL OF THE AMERICAN OSTEOPATHIC ASSOCIATION. MAY 1968, vol. 67, no. 9, May 1968 (1968-05), pages 1051-1053, XP009059014 ISSN: 0098-6151
- GUIN L W ET AL: "ELECTROPHORETIC CHARACTERIZATION OF PLASMA RNA" BIOCHEMICAL MEDICINE, vol. 13, no. 3, 1975, pages 224-230, XP002360002 ISSN: 0006-2944
- STROUN M ET AL: "Presence of RNA in the nucleoprotein complex spontaneously released by human lymphocytes and frog auricles in culture." CANCER RESEARCH. OCT 1978, vol. 38, no. 10, October 1978 (1978-10), pages 3546-3554, XP009058953 ISSN: 0008-5472
- ALLOUCHE M ET AL: "Expression of basic fibroblast growth factor (bFGF) and FGF-receptors in human leukemic cells." LEUKEMIA : OFFICIAL JOURNAL OF THE LEUKEMIA SOCIETY OF AMERICA, LEUKEMIA RESEARCH FUND, U.K. JAN 1995, vol. 9, no. 1, January 1995 (1995-01), pages 77-86, XP009058965 ISSN: 0887-6924
- RICCHIUTI V ET AL: "Expression of cardiac troponin T mRNA in skeletal muscle from patients with end stage renal disease and muscular dystrophy" CLINICAL CHEMISTRY, vol. 45, no. 6 PART 2, June 1999 (1999-06), pages A144-A145, XP009058963 & 51ST ANNUAL MEETING OF THE AMERICAN ASSOCIATION OF CLINICAL CHEMISTRY; NEW ORLEANS, LOUISIANA, USA; JULY 25-29, 1999 ISSN: 0009-9147
- RICCHIUTI VINCENT ET AL: "RNA expression of cardiac troponin T isoforms in diseased human skeletal muscle" CLINICAL CHEMISTRY, vol. 45, no. 12, December 1999 (1999-12), pages 2129-2135, XP002360003 ISSN: 0009-9147
- RICCHIUTI VINCENT ET AL: "Cardiac troponin I and T alterations in hearts with severe left ventricular remodeling" CLINICAL CHEMISTRY, vol. 43, no. 6 PART 1, 1997, pages 990-995, XP002360007 ISSN: 0009-9147
- ROSENZWEIG A ET AL: "PRECLINICAL DIAGNOSIS OF FAMILIAL HYPERTROPHIC CARDIOMYOPATHY BY GENETIC ANALYSIS OF BLOOD LYMPHOCYTES" NEW ENGLAND JOURNAL OF MEDICINE, THE, MASSACHUSETTS MEDICAL SOCIETY, WALTHAM, MA, US, vol. 325, no. 25, 19 November 1981 (1981-11-19), pages 1753-1760, XP001119222 ISSN: 0028-4793
- SPIEGELMAN S: "THE DEVELOPMENT AND USE OF AN EXTRACELLULAR RNA REPLICATING SYSTEM" GOWANS, J.L., R.A. GREGORY, D.M. HUME, JERARD HURWITZ, HENRY S. KAPLAN, ROBERT L. SINSHEIMER, S. SPIEGELMAN AND JACK L. STROMINGER. THE HARVEY LECTURES. NO. 64. DELIVERED UNDER THE AUSPICES OF THE HARVEY SOCIETY OF NEW YORK, 1969-1969. XIV + 428P. IL, 1970, pages 1-67, XP001207699
- DASI F. ET AL.: 'Real-time quantification in plasma of human telomerase reverse transcriptase (hTERT) mRNA: a simple blood test to monitor disease in cancer patients' LABORATORY INVESTIGATION vol. 81, no. 5, 2001, pages 767 - 769, XP002963619
- KOPRESKI M.S. ET AL.: 'Detection of tumor messenger RNA in the serum of patients with malignant melanoma' CLINICAL CANCER RESEARCH vol. 5, August 1999, pages 1961 - 1965, XP002963620

## Description

### BACKGROUND OF THE INVENTION

The present invention covers 12 embodiments as follows:
1. A method for detecting, diagnosing or monitoring, an injury of an organ in a human, the method comprising the step of detecting qualitatively or quantitatively extracellular human RNA in a bodily fluid previously obtained from said human, wherein said human RNA is detected by assaying for said human RNA or cDNA derived therefrom, in a qualitative or a quantitative fashion, wherein the injury of an organ is traumatic or ischemic injury of the brain or heart.
2. The method of 1, wherein traumatic or ischemic injury of the brain or heart is a stroke, myocardial infarction, myocardial ischemia, ischemic brain injury, hypoxia of the brain, or head trauma.
3. The method of 1 or 2, wherein the bodily fluid is selected from blood, blood plasma, urine, cerebrospinal fluid or serum.
4. A method for detecting extracellular human RNA in blood, blood plasma or serum, or other bodily fluid, all of them, blood, blood plasma, serum, or other bodily fluid, previously obtained from a human, wherein said RNA is associated with an injury of an organ of said human, the method comprising the steps of:
   a) extracting RNA from blood, blood plasma, serum, or other bodily fluid from a human, wherein a portion of said extracted RNA comprises an extracellular human RNA associated with an injury of an organ of said human;
   b) *in vitro* amplifying or signal amplifying a fraction of the extracted RNA or cDNA derived therefrom in a qualitative or quantitative fashion using primers or probes specific for a human nucleic acid sequence of said extracellular human RNA, or cDNA derived therefrom, to produce an amplified product or signal;
   c) detecting the amplified product or signal produced thereby,
   wherein the injury of an organ is traumatic or ischemic injury of the brain or heart.
5. The method of 4, wherein said extracellular human RNA is derived from heart, or brain of said human.
6. The method according to 4 or 5, whereby an injury of an organ is detected, diagnosed or monitored.
7. The method according to 4, 5 or 6, wherein said organ is heart or brain of the human.
8. The method according to any of 4 to 7, wherein the human RNA translates a protein that has a deleterious effect upon cells or tissues within the human.
9. A method of detecting extracellular human RNA obtained from plasma or serum from a human, said human RNA being associated with an injury of an organ of said human, the method comprising the steps of:
   a) extracting RNA from blood plasma or serum from a human, wherein a portion of said RNA comprises an extracellular human RNA associated with an injury of an organ of said human;
   b) hybridizing a portion of said extracellular human RNA associated with an injury of an organ of said human, or cDNA derived therefrom, to a primer, probe, or solid substrate, wherein the primer, probe, or solid substrate is specific for human nucleotide sequences of said extracellular human RNA, or cDNA derived therefrom;
   c) detecting the hybridized RNA or cDNA,
   wherein the injury of an organ is a traumatic or ischemic injury of the brain or heart.
10. The method of 4 or 9, wherein said human RNA is cardiac troponin T mRNA, cardiac troponin I mRNA, beta-myosin heavy chain mRNA, acidic fibroblast growth factor mRNA, or Par-4 mRNA.
11. The method of 9, wherein said extracellular human RNA is derived from heart or brain.
12. The method of 4 or 9, wherein said injury of an organ is one from a group comprising myocardial infarction, myocardial ischemia, congestive heart failure, cardiomyopathy, stroke, ischemic brain injury, hypoxic conditions of the brain, and head trauma.

This disclosure illustrates methods for diagnosing, detecting, evaluating, and monitoring non-neoplastic pathologic conditions and diseases within an animal, preferably a human. Said pathologic conditions and diseases include, inter alia, pathologic conditions and diseases affecting specific body organs and those affecting multiple organs or bodily organ systems, and those pathologic conditions that are associated with disease or injury, or that are predictive for a disease or that can ultimately result in a disease. As set forth herein, the disclosure illustrates methods for detecting mammalian ribonucleic acid (RNA) in said animal's blood plasma, serum, or other bodily fluid. The methods thereby enable evaluation of gene expression that is associated with, consequent to, or predictive of pathologic conditions and diseases or cellular injury and trauma. The disclosure also illustrates methods that permit cellular response and recovery to pathologic conditions and disease as well as cellular injury to be monitored. The disclosure thereby illustrates methods for evaluating and monitoring response to specific therapies for said pathologic conditions, diseases, and injuries. The disclosure also specifically illustrates methods for evaluating and monitoring non-hematopoietic or non-hematological cells and tissues that are terminally differentiated. In these methods, extracellular RNA derived from said cells and tissues is detected in a bodily fluid. The disclosure also illustrates the diagnosing, detecting, evaluating, and/or monitoring of pathologic conditions and diseases affecting non-proliferating cells and tissues, such as those of the heart, brain and muscle. The approach is thus particularly advantageous for evaluating pathologic conditions and diseases of the cardiovascular system, the nervous system and of skeletal muscles. The disclosure further illustrates the detection of non-neoplastic cells and tissues that are proliferating normally or consequent to disease or injury. The disclosure illustrates detection of extracellular mammalian RNA associated with non-neoplastic disease that is not transcribed from a fragile site or does not contain viral or bacterial nucleic acid sequences.

While the etiologies of non-neoplastic pathologic conditions and diseases are varied, the pathologic process is often characteristically associated with intracellular production or over-production, or escape or release, of specific proteins from the cell that can characterize the cell. Such proteins may be involved in cellular adaptive responses, or be indicative of cellular injury, or reflect the production of proteins associated with the disease state itself. Furthermore, proteins normally associated with a cell or tissue's metabolism may be overproduced within a cell, or be secreted from the cell, or be inappropriately released from the cell. In clinical practice, detection of proteins in blood and other bodily fluids has been utilized in the diagnosis and monitoring of disease. However, not all such proteins may be detectable in blood or bodily fluids, often because the protein is either not secreted or released from the cell, or exists in blood at levels below limits of detection for a given stage of disease, particularly at early or subclinical stages of disease. There thus exists a need for new methods that provide for the analysis of cellular gene expression in a more sensitive manner.

Ribonucleic acid (RNA) is essential for producing cellular proteins, and detecting and monitoring mammalian RNA can be used to assess cellular gene expression. Furthermore, since RNA and deoxyribonucleic acid (DNA) can be hybridized and amplified in a qualitative or quantitative manner using nucleic acid amplification methods, detection of RNA can be performed with high sensitivity. Although the prior art contained sporadic reports suggesting that RNA might be detected in plasma and serum *(e.g.,* Wieczorek et al., 1985 Proc Natl Acad Sci USA 82: 3455-3459; Wieczorek et al., 1987 Cancer Res. 47: 6407-6412; Wieczorek et al., 1989 Schweiz med Wschr 119: 1342-1343; Kamm and Smith, 1972 Clin. Chem. 18: 519-522), until recently it was unknown whether specific RNA species existed in plasma or serum with sufficient integrity to be amplified and detected. Co-owned U.S. Patent No. 6,329,179 B1, provide methods for detecting extracellular tumor RNA in blood plasma, serum, and bodily fluids. After the priority date of the cho-owned application, several authors have confirmed that tumor RNA can be amplified from plasma or serum (Kopreski et al., 1999 Clin. Cancer Res. 5: 1961-1965; Chen et al., 2000 Clin. Cancer Res. 6: 3823-3826; Dasi et al., 2001 Lab. Invest. 81: 767-769; Hasselmann et al., 2001 Oncology Reports 8: 115-118; Kopreski et al., 2001 Clin. Chem. 47: 362, abstract 9; Fleishhacker et al., 2001 Clin. Chem. 47: 369, abstract 48; Reinhold et al., 2001 Clin. Chem. 47: 369, abstract 50; Gocke et al., 2001 Clin. Chem. 47: 369, abstract 51), and further that fetal RNA is detectable in maternal plasma (Poon et al., 2001 Clin. Chem. 47: 363, abstract 11). These findings are notable since it is well established in the art that ribonucleases present in blood rapidly degrade mammalian RNA (Reddi and Holland, 1976 Proc Nat Acad Sci USA 73: 2308-2310), and further that one consequently can not amplify free RNA from plasma or serum following cellular lysis (Komeda et al., 1995 Cancer 75: 2214-2219; Pfleiderer et al., 1995 Int. J. Cancer 64: 135-139). Mammalian RNA has also been demonstrated in sera in association with viral nucleic acid, and fragile sites, such as in association with hematologic cancer cells (Urnovitz et al., 1999 Clin. Diag. Lab. Immunology 6: 330-335; Urnovitz, U.S. Patent Serial No. 6,344,317, WO98/14617). Since the etiology and physiology of extracellular ' RNA remains unknown, detection of extracellular RNA in non-virally mediated, non-neoplastic disease processes, and particularly from non-hematological cells and tissues that include non-proliferating tissues and terminally differentiated cells and tissues of diseased or injured solid organs, was both unknown and unexpected.

Neoplasia is characterized by pathophysiologic processes that often differ from those of non-neoplastic disease. Similarly, fetal development may be viewed as a proliferative process of cells undergoing differentiation characterized by physiologic processes that often differ from those occurring in non-neoplastic disease. It was unknown in the art that extracellular mammalian RNA derived from non-neoplastic solid organ tissue could be detected in the blood plasma, serum, or other bodily fluids of individuals with disease at levels higher than present in the blood plasma or serum or bodily fluid of healthy individuals. This is particularly true for non-neoplastic, non-virally mediated RNA specific to the non-proliferating, terminally differentiated non-hematopoietic or non-hematological cells and tissues of the heart and brain.

### SUMMARY OF THE INVENTION

The present invention provides methods for diagnosing, evaluating, or monitoring within a human, the existence of an injury, which is traumatic or ischemic brain or heart injury. In preferred embodiments, the method comprises the step of detecting extracellular mammalian RNA in a bodily fluid of an animal, preferably blood and most preferably blood plasma or serum, urine, effusions, ascites, saliva, cerebrospinal fluid, cervical secretions, vaginal secretions, endometrial secretions, gastrointestinal secretions, sputum and bronchial secretions, and/or associated lavages, wherein said RNA is present in the bodily fluid of an animal with a cellular injury, and not present in the bodily fluid of a healthy animal, or wherein said RNA is present in a bodily fluid of an animal with cellular injury in quantitative amounts that are greater than are present in the bodily fluid of a healthy animal.

The invention provides methods for amplifying and detecting extracellular mammalian RNA associated with said injury in blood, more preferably in blood plasma or serum, or in other bodily fluids, the method comprising the steps of extracting RNA from said bodily fluid, *in vitro* amplifying or signal amplifying a fraction of the extracted RNA or cDNA derived therefrom, either qualitatively or quantitatively, and detecting the amplified product or signal produced thereby.

In a preferred embodiment, the RNA is derived from a non-hematopoietic or non-hematological cell or tissue. In one aspect of this embodiment, the RNA is derived from a non-proliferating cell or tissue. In a second aspect, the RNA is derived from a terminally-differentiated cell or tissue. In a third aspect, the RNA does not contain viral nucleic acid sequences. In a fourth aspect, the RNA is not derived from transcription of a fragile site.

The invention in the above embodiments 1-12 provides methods for detecting organ-specific or tissue-specific extracellular mammalian RNA present in plasma, serum, and/or other bodily fluid by hybridization, wherein the RNA is derived from specific non-neoplastic non-hematopoietic or non-hematological cells and/or tissue from a human, or cDNA derived therefrom, to a specific primer, probe, solid substrate or bioelectrical interface, the method comprising the steps of extracting RNA from said bodily fluid, and hybridizing a portion of the extracted RNA or cDNA derived therefrom to a specific primer, probe, solid substrate, or bioelectrical interface consisting of oligonucleotide sequences complimentary to RNA or cognate cDNA from specific, non-neoplastic, non-hematopoietic or non-hematologic cells and/or tissue.

In preferred embodiments, the extracellular RNA detected using the methods of this invention are not amplified. In one aspect of this embodiment, the RNA does not contain viral nucleic acid sequences. In a second aspect of this embodiment, the RNA is not transcribed from a fragile site. In a third aspect of this embodiment, the cells or tissues are terminally differentiated. In a fourth aspect of this embodiment, the cells or tissues are non-proliferating.

The invention in the above embodiments 1-12 provides methods for detecting and monitoring mammalian RNA or cDNA derived therefrom, in blood, preferably blood plasma, serum, and other bodily fluids from an animal, most preferably a human, that is associated with non-hematopoietic or non-hematological cells or tissue affected by a non-neoplastic disease or injury, wherein the method comprises the steps of extracting RNA from said bodily fluid, *in vitro* amplifying or signal amplifying a fraction of the extracted RNA or cDNA derived therefrom, either qualitatively or quantitatively, and detecting the amplified product or signal produced thereby.

In one aspect of this embodiment, the RNA does not contain viral or retroviral nucleic acid sequences. In a second aspect of this embodiment, the RNA is not transcribed from a fragile site. In a third aspect of this embodiment, the cells or tissues are terminally differentiated. In a fourth aspect of this embodiment, the cells or tissues are non-proliferating.

The invention in the above embodiments 1-2 provides methods for detecting and monitoring mammalian RNA or cDNA derived therefrom, in blood, preferably blood plasma or serum, or other bodily fluid from an animal, most preferably a human, that is derived from non-neoplastic cells or tissues, wherein said mRNA produces a protein that has a consequent deleterious effect upon other differing non-neoplastic cells or tissues, thereby resulting in a disease or pathologic condition of the cells or tissues or their organ(s) and organ system(s) thereby deleteriously affected. In this embodiment, the method comprises the steps of extracting RNA from blood plasma, serum, or other bodily fluid, in *vitro* amplifying or signal amplifying the RNA or cDNA derived therefrom, either qualitatively or quantitatively, and detecting the amplified product or signal produced thereby.

In one aspect of this embodiment, the RNA does not contain viral or retroviral nucleic acid sequences. In another aspect of this embodiment, the RNA is not transcribed from a fragile site: In another aspect of this embodiment, the cells or tissues are terminally differentiated. In another aspect of this embodiment, the cells or tissues are non-proliferating.

The invention in the above embodiments provides methods for detecting and monitoring mammalian RNA or cDNA derived therefrom, in blood, most preferably plasma or serum, and/or other bodily fluid from an animal, most preferably a human, that is associated with non-neoplastic, terminally differentiated non-hematopoietic or non-hematological cells or tissues, including either healthy or diseased tissues, the method comprising the steps of extracting RNA from said bodily fluid, *in vitro* amplifying or signal amplifying the extracted RNA or cDNA derived therefrom either qualitatively or quantitatively, and detecting the amplified product or signal produced thereby.

In one aspect of this embodiment, the present invention provides methods for detecting human RNA associated with non-hematopoietic or non-hematological cells and tissues that are characteristic of specific tissue(s) or organ(s) or organ system(s), either diseased or healthy. In this aspect, the methods of the invention comprise the steps of extracting RNA from blood, most preferably blood plasma or serum, or other bodily fluid, *in vitro* amplifying or signal amplifying RNA comprising said extracted RNA or cDNA derived therefrom, associated with non-hematological cells and tissues of specific organ(s) or organ system(s), either qualitatively or quantitatively, and then detecting the amplified product or signal. The cells and tissues are those of the heart or cardiovascular system, and the cells and tissues are those of the brain or nervous system.

In a second aspect of this embodiment, the invention provides methods for detecting mammalian RNA from a non-heenatopoietic or non-hematological, non-proliferative tissue in a bodily fluid such as blood, blood plasma, serum, or cerebrospinal fluid. In this aspect, the methods of the invention comprise the steps of extracting human RNA from said bodily fluid, *in vitro* amplifying or signal amplifying a fraction of the extracted RNA or cDNA derived therefrom, comprising said extracted RNA associated with a non-hematopoietic or non-hematological non-proliferative tissue, either qualitatively or quantitatively, and then detecting the amplified product or signal thereby. The non-proliferative tissue is heart tissue, preferably cardiac muscle tissue or the non-proliferative tissue is brain tissue, preferably neural tissue.

In preferred embodiments of the inventive methods, extracellular human RNA is extracted from a bodily fluid such as whole blood, blood plasma or serum, or cerebrospinal fluid, using an extraction method such as gelatin extraction method; silica, glass bead, or diatom extraction method; guanidinium thiocyanate acid-phenol based extraction methods; guanidinium thiocyanate acid based extraction methods; methods using centrifugation through cesium chloride or similar gradients; phenol-chloroform based extraction methods; or other commercially available RNA extraction methods. Extraction may further be performed using probes that specifically hybridize to specific RNA, including probes attached to solid substrates or to magnetic beads or similar particles.

In preferred embodiments of the inventive methods, human RNA or cDNA derived therefrom, or a signal derived therefrom, is amplified using an amplification method such as reverse transcriptase polymerase chain reaction (RT-PCR); ligase chain reaction; DNA signal amplification; amplifiable RNA reporters; Q-beta replication; transcription-based amplification; isothermal nucleic acid sequence based amplification; self-sustained sequence replication assays; boomerang DNA amplification; strand displacement activation; cycling probe technology; or any combination or variation thereof.

In preferred embodiments of the inventive methods, detecting an amplification product of the human RNA or cDNA derived therefrom or signal derived therefrom is accomplished using a detection method such as gel electrophoresis; capillary electrophoresis; conventional enzyme-linked immunosorbent assay (ELISA) or modifications thereof, such as amplification using biotinylated or otherwise modified primers; nucleic acid hybridization using specific, detectably-labeled probes, such as fluorescent-, radioisotope-, or chromogenically-labeled probe; laser-induced fluorescence detection; Northern blot analysis; Southern blot analysis; electrochemiluminescence; reverse dot blot detection; and high-performance liquid chromatography.

In particularly preferred embodiments of the inventive methods, human RNA is converted to cDNA using reverse transcriptase following extraction of RNA from a bodily fluid and prior to amplification.

The methods of the embodiments 1-12 of the invention are advantageously used for providing a diagnosis for an injury as described above. The methods of the invention are particularly useful for providing a diagnosis or monitoring of, injuries of the heart and cardiovascular system. Cardiovascular disease is one of the most common potentially life-threatening non-neoplastic human diseases throughout the world. The methods of the invention enable diagnosis, detection, evaluation, and monitoring of cardiovascular disease, including but not limited to diseases and pathologic conditions of the heart such as myocardial infarction, myocardial ischemia, coronary insufficiency, congestive heart failure, cardiomyopathy, atherosclerosis, intimal hyperplasia, and cardiac transplant rejection, and conditions associated with angina, and conditions and diseases associated with atherosclerosis and intimal hyperplasia or smooth muscle cell hyperplasia, and pathologic conditions and diseases associated with hypertension. The methods of the invention provide qualitative or quantitative detection of extracellular RNA in the blood plasma, serum, or other bodily fluid of a human, and wherein the RNA is associated with cardiovascular disease or pathologic conditions, including those of the heart and those of the vasculature, or with cells and tissues of the heart, arteries, and veins. Extracellular RNA associated with cardiovascular disease and pathologic conditions and/or injury includes, but is not limited to cardiac troponin T RNA (cTnT RNA), cardiac troponin I RNA (cTnI RNA), beta-myosin heavy chain RNA, acidic fibroblast growth factor RNA (heparin binding growth factor-1), basic fibroblast growth factor RNA, and platelet-derived growth factor-A and B RNA (PDGF-A RNA and PDGF-B RNA). It is to be understood that these RNA species provide examples and not limitation of the invention.

The methods of the invention are further particularly useful for providing a diagnosis for, injuries as defined above. The methods of the invention are applicable to injuries of the brain as defined above. The methods of the invention enables diagnosing, detecting, evaluating, and monitoring of diseases and conditions of the central nervous system, including but not limited to stroke, ischemic brain injury, hypoxic conditions of the brain, head trauma, multiple sclerosis, Alzheimer's disease, encephalopathies, and neurodegenerative diseases. The inventive methods provide qualitative or quantitative detection of extracellular mammalian RNA in the cerebrospinal fluid or other bodily fluid of an animal, most preferably a human, wherein the RNA is associated with a neurologic disease or condition such as injury or trauma, or with cells and tissues of the central nervous system. Extracellular RNA associated with neurologic disease and/or neurologic injury includes, but is not limited to the mutated presenilin 1 gene (PS1) RNA, mutated presenilin 2 gene (PS2) RNA, and Par 4 (prostate apoptosis response - 4) RNA. It is to be understood that these RNA species provide examples and not limitation of the invention.

The methods illustrate usefulness in non-neoplastic diseases and pathologic conditions affecting other solid organs and organ systems, such as those of the gastrointestinal system, the genitourinary system, the endocrine system, the respiratory system, the musculoskeletal system, and the skin. In these illustrates applications the method illustrates qualitative or quantitative detection of extracellular mammalian RNA in the blood plasma, serum, or other bodily fluid of an animal, most preferably a human, wherein the RNA is associated with a disease or pathologic condition of said organ or organ system and/or its cells and tissues. For example, cardiac troponin T mRNA (cTnT mRNA) is further detectable is some cases of skeletal muscle disease or pathologies such as Duchenne muscular dystrophy, polymyositis, and myopathy induced from end-stage renal disease.

In certain preferred embodiments of the methods of the invention, mammalian RNA associated with non-neoplastic, non-hematopoietic or non-hematological cells or tissue, or cDNA derived therefrom, is amplified in a quantitative manner, thereby enabling the quantitative comparison of said RNA or cDNA present in a bodily fluid such as blood plasma, serum, or cerebrospinal fluid from a non-pregnant animal, preferably a human. Here, the amount of said RNA detected in bodily fluid from a particular individual animal is compared with a range of amounts of said RNA detected in said bodily fluid in healthy populations of animals, wherein increased amounts of RNA in said bodily fluid from the particular individual animal in comparison to healthy animals is indicative of a disease or pathologic condition, or is a predictive indicator of a disease or pathologic condition. The non-neoplastic, non-hematological cells or tissue may the terminally differentiated cells or tissue, or non-proliferative cells or tissue. In particularly preferred embodiments the cells or tissue are those of the heart, brain or muscle_{.}

The methods disclosed herein further illustrate ways to identify animals, most preferably humans, having non-neoplastic disease or pathologic conditions, thereby permitting rational, informed treatment options to be used for making therapeutic decisions.

Another advantageous use for the methods disclosed herein is to illustrate a marker for assessing the adequacy of therapy, or for determining whether additional or more advanced or efficacious therapy is required. The disclosure therefore illustrates methods for developing a prognosis in such patients.

Another advantageous use for the methods disclosed herein is to illustrate for the screening of individuals as to determine their predisposition for a disease or pathologic condition, and further to determine their need for further diagnostic evaluation and/or for preventive therapy.

In a particularly preferred embodiment, the present invention provides methods for detecting extracellular cardiac troponin T mRNA or cardiac troponin I mRNA and their isoforms in blood or blood factions, including plasma and serum, or in other bodily fluid, in a human. As provided herein, the methods comprise the steps of extracting RNA from blood, blood plasma, serum, or other bodily fluid, in vitro amplifying cardiac troponin T mRNA or cDNA derived therefrom, and/or *in vitro* amplifying cardiac troponin I RNA or cDNA derived therefrom, either qualitatively or quantitatively, and detecting the amplified product of cardiac troponin T mRNA or cDNA and/or of cardiac troponin I mRNA or cDNA.

In a first aspect of this embodiment, the present invention provides methods for detecting cardiac troponin T mRNA and/or cardiac troponin I mRNA in blood or blood fractions, including plasma and serum, or other bodily fluid in a human as a method for detecting, diagnosing, or monitoring, for a disease or pathologic condition of the heart such as clinical or subclinical myocardial infarction or ischemic heart disease or coronary insufficiency.

In a particularly preferred embodiment, the present invention provides a method for detecting extracellular beta-myosin heavy chain mRNA in blood or blood fractions, including plasma and serum, or in other bodily fluid, in a human, the method comprising the steps of extracting RNA from blood, blood plasma, serum, or other bodily fluid, *in vitro* amplifying beta-myosin heavy chain mRNA or cDNA derived therefrom, either qualitatively or quantitatively, and detecting the amplified product of beta-myosin heavy chain mRNA or cDNA.

In a first aspect of this embodiment, the present invention provides methods for detecting beta-myosin heavy chain mRNA in blood or blood fractions, including plasma and serum, or other bodily fluid in a human as a method for detecting, diagnosing, or monitoring, for a disease or pathologic condition of the heart such as those associated with myocardial injury.

In a particularly preferred embodiment, the present invention provides a method for detecting extracellular acidic fibroblast growth factor mRNA (heparin-binding growth factor-1 mRNA) and/or extracellular basic fibroblast growth factor mRNA in blood or blood fractions, including blood plasma and serum, or other bodily fluid, in a human, the method comprising the steps of extracting RNA from blood, blood plasma, serum, or other bodily fluid, *in vitro* amplifying acidic fibroblast growth factor mRNA or cDNA derived therefrom, and/or basic fibroblast growth factor mRNA or cDNA derived therefrom, either qualitatively or quantitatively, and detecting the amplified product of acidic fibroblast growth factor mRNA or cDNA and/or basic fibroblast growth factor mRNA or cDNA.

In a first aspect of this embodiment, the present invention provides methods for detecting acidic fibroblast growth factor mRNA or basic fibroblast growth factor mRNA in blood or blood fractions, including blood plasma and serum, or other bodily fluid in a human, as a method for detecting, diagnosing, or monitoring, for a disease or pathologic condition of vascular smooth muscle, most preferably atherosclerosis and/or intimal hyperplasia.

In a particularly preferred embodiment, the present invention provides a method for detecting extracellular prostate apoptosis response - 4 (Par-4) mRNA in cerebrospinal fluid, blood or blood fractions including plasma and serum, or other bodily fluid, in a human, the method comprising the steps of extracting RNA from cerebrospinal fluid, blood, plasma, serum, or other bodily fluid, *in vitro* amplifying Par-4 mRNA or cDNA derived therefrom, either qualitatively or quantitatively, and detecting the amplified product of Par-4 mRNA or cDNA.

In a first aspect of this embodiment, the present invention provides methods for detecting Par-4 mRNA in cerebrospinal fluid, blood or blood fractions including plasma and serum, or other bodily fluid in a human as a method for detecting, diagnosing, or monitoring, for a disease or pathologic condition or injury of the brain. In a particularly advantageous use of the invention, the disease or pathologic condition or injury of the brain is stroke, ischemia of the brain, hypoxia of the brain, traumatic brain injury, and/or neurodegenerative diseases.

The disclosure also illustrates diagnostic kits for use in the practice of the methods of the invention, specifically for the detection, diagnosis, monitoring, prognosticating, or predicting of non-neoplastic disease or pathologic disease or injury, wherein the diagnostic kit provides reagents for the extraction of mammalian RNA from plasma, serum, or other bodily fluid, and primers or probes used in the detection of the extracted RNA or cDNA derived therefrom

Specific preferred embodiments of the present invention will become evident from the following more detailed description of certain preferred embodiments and the claims.

### DETAILED DESCRIPTION OF THE INVENTION

The disclosure illustrates methods for diagnosing, evaluating, predicting within, or monitoring an animal, most preferably a human, for non-neoplastic diseases or pathologic conditions or injury by detecting extracellular mammalian RNA associated with said disease or pathologic condition or injury, such as but not limited to RNA derived from non-neoplastic, non-hematopoietic or non-hematological cells or tissue; RNA from terminally differentiated cells or tissue; RNA from non-proliferative cells, and/or RNA specific to cells or tissues of an organ(s) or organ system(s), wherein the RNA is detected in a bodily fluid of said animal, preferably blood and most preferably blood plasma and serum as well as in other bodily fluids, preferably cerebrospinal fluid, urine, saliva, effusions including pleural effusion, pericardial effusion, and joint effusion, ascites, cervical secretions, vaginal secretions, endometrial secretions, gastrointestinal secretions, sputum and bronchial secretions, and fluids associated with tissue lavages.

The disclosure further illustrates a method for detecting and/or monitoring mammalian RNA in blood plasma, serum, and/or bodily fluid from an animal, most preferably a human, or cDNA derived therefrom, that is associated with non-hematopoietic or non-hematological cells or tissue affected by a non-neoplastic disease or injury, wherein the method comprises the steps of extracting RNA from blood plasma, serum or other bodily fluid, *in vitro* amplifying or signal amplifying a fraction of the extracted RNA or cDNA derived therefrom, either qualitatively or quantitatively, and detecting the amplified product or signal of the RNA or cDNA derived therefrom.

The disclosure illustrates the detection of mammalian RNA that does not contain viral or retroviral nucleic acid sequences within its own sequence. The disclosure further provides for the detection of mammalian RNA that is not transcribed from a fragile site of genomic DNA, wherein a fragile site is a locus that is a frequent site of DNA strand breakage. Thus the disclosure further illustrates the detection of RNA that is transcribed from wild-type genomic DNA, in addition to detection of RNA transcribed from a mutated, deleted, translocated, methylated, or otherwise altered genomic DNA. The disclosure illustrates the detection of messenger RNA, in addition to non-messenger RNA species such as ribosomal RNA, transfer RNA, ribonucleoprotein, and RNA transcribed from non-nuclear DNA.

The disclosure further illustrates a method for detecting and/or monitoring mammalian RNA in blood plasma, serum, and/or other bodily fluid from an animal, most preferably a human, or cDNA derived therefrom, that is associated with non-neoplastic, non-hematopoietic or non-hematological terminally differentiated cells or tissues or non-proliferative cells or tissues, including either healthy or diseased tissues, the method comprising the steps of extracting RNA from blood plasma, serum, or other bodily fluid, in vitro amplifying or signal amplifying a fraction of the extracted RNA or cDNA derived therefrom either qualitatively or quantitatively, and detecting the amplified product or signal of RNA or cDNA derived therefrom, wherein the amplified product or signal is produced from an mRNA that is specific for non-neoplastic, non-hematopoietic or non-hematological terminally differentiated cells or tissues or non-proliferative cells or tissues.

The disclosure further illustrates methods for detecting and/or monitoring mammalian RNA associated with non-neoplastic, non-hematopoietic or non-hematological cells and tissues that are characteristic of specific tissue(s) and/or organ(s) and/or organ system(s), either diseased or healthy, the methods comprising the steps of extracting RNA from blood plasma, serum, or other bodily fluid, *in vitro* amplifying or signal amplifying a fraction of the extracted RNA or cDNA derived therefrom either qualitatively or quantitatively, and detecting the amplified product or signal of RNA or cDNA derived therefrom, wherein the amplified product or signal is produced from an mRNA that is specific for non-neoplastic, non-hematopoietic or non-hematological terminally differentiated cells or tissues or non-proliferative cells or tissues that are characteristic of specific tissue(s) and/or organ(s) and/or organ system(s), either diseased or healthy.

The disclosure further illustrates methods for detecting and/or monitoring mammalian RNA in blood, blood plasma, serum, and/or other bodily fluid from an animal, most preferably a human, or cDNA derived therefrom, that is derived from non-neoplastic cells or tissues, when said RNA produces a protein that has a consequent deleterious effect upon other differing non-neoplastic cells or tissues, thereby resulting in a disease or pathologic condition of the cells or tissues or their organ(s) and organ system(s) deleteriously affected, wherein the method comprises the steps of extracting RNA from blood plasma, serum, or other bodily fluid, in *vitro* amplifying or signal amplifying a fraction of the extracted RNA or cDNA derived therefrom, either qualitatively or quantitatively, and detecting the amplified product or signal of the RNA or cDNA derived therefrom. For example, the methods may be used to detect within a bodily fluid mRNA associated with production of lipoproteins, wherein said mRNA is derived from cells of the liver, and wherein said protein has a deleterious effect upon the cells of the vascular system such as the arteries, thereby resulting in atherosclerosis.

In the methods extracellular mammalian RNA may be extracted from a bodily fluid of an animal, most preferably a human. From this extracted RNA, mRNA associated with a non-neoplastic disease or pathologic condition or injury, or derived from specific non-neoplastic cells, tissues, or organs of the animals, is then amplified, either after conversion into cDNA or directly, using in *vitro* amplification methods in either a qualitative or quantitative manner, or by amplification of a signal associated with the mRNA or cDNA derived therefrom in a qualitative or quantitative manner. The amplified product is then detected in either a qualitative or quantitative manner.

In additional aspects , organ-specific or tissue-specific or tissue-identifiable extracellular mammalian RNA present in a bodily fluid, most preferably blood plasma and serum, that is derived from specific non-neoplastic non-hematopoietic or non-hematological cells or tissue of an animal, most preferably a human, or cDNA derived therefrom, is hybridized to a specific primer, probe, solid substrate, or bioelectrical interface, the method comprising the extraction of RNA from a bodily fluid, most preferably plasma or serum, and hybridizing the RNA or cDNA derived therefrom to a specific primer, probe, solid substrate, or bioelectrical interface that consists of oligonucleotide sequences complimentary to RNA from specific non-neoplastic non-hematological cells and/or tissue, or cDNA derived therefrom. The disclosure thereby illustrates the products of the hybridization.

In the practice of the methods, extracellular mammalian RNA may be extracted from any bodily fluid, including but not limited to whole blood, plasma, serum, cerebrospinal fluid, urine, saliva, effusions including pleural effusion, pericardial effusion, and joint effusions, ascites, cervical secretions, vaginal secretions, endometrial secretions, gastrointestinal secretions, sputum and bronchial secretions, and fluids associated with tissue lavages, using, *for example,* extraction methods described in co-owned U.S. Patent No. 6,329,179 B1 . In the practice extracellular mammalian RNA may be extracted from the bodily fluid using methods such as, but not limited to, gelatin extraction method; silica, glass bead, or diatom extraction method; guanidinium thiocyanate acid-phenol based extraction methods; guanidinium thiocyanate acid based extraction methods; methods using centrifugation through cesium chloride or similar gradients; phenol-chloroform based extractions methods; and/or other available RNA extraction methods, as are known in the art for use in extraction of intracellular RNA, including commercially available RNA extraction methods, *for example,* by using or adapting or modifying the methods of Boom et al. (1990 J. Clin. Microbiology 28: 495-503); Cheung et al. (1994 J. Clin. Microbiology 32: 2593-2597); Boom et al. (1991 J. Clin. Microbiology 29: 1804-1811); Chomczynski and Sacchi (1987 Analytical Biochem. 162:156-.159); Chomczynski, (1993 Biotech. 15: 532-537); Chomczynski and Mackey (1995 Biotechniques 19: 942-945); Chomczynski and Mackey (1995 Analytical Biochem. 225: 163-164); Chirgwin et al. (1979 Biochem. 18: 5294-5299); Fournie et al. (1986 Analytical Biochem. 158: 250-256); and further as described in co-owned U.S. Patent No. 6,329,179 B1, It is further to be understood that any RNA extraction method that has demonstrated suitability for the extraction of tumor-derived or tumor-associated RNA from plasma or serum or other bodily fluid is hereby recognized as being suitable for the extraction of non-neoplastic mammalian RNA from bodily fluid.

The extraction method used for extraction of extracellular mammalian RNA may be a commercially available extraction method suitable for extraction of intracellular RNA, *for example,* TRIzol ^{™} (Life Technologies); Trisolv ^{™} (BioTecx Laboratories); ISOGEN ^{™} (Nippon Gene); RNA Stat ^{™} (Tel-test); TRI Reagent ^{™} (Sigma); SV Total RNA Isolation System (Promega); RNeasy Mini Kit (Qiagen); Perfect RNA: Total RNA Isolation Kit (Five Prime-Three Prime Inc., Boulder, Colorado); or similar commercially available kit, wherein extraction of RNA may be performed according to manufacturer's directions, adapted to the bodily fluid.

RNA may be extracted from a bodily fluid using a probe or probes that specifically hybridize to specific RNA species, such as but not limited to probes attached to solid substrates or probes attached to magnetic beads or particles, or probes wherein upon hybridization to a nucleic acid, an electrical gradient or magnetic gradient or density gradient can thereby enable extraction and/or separation of specific RNA species from the remainder of bodily fluid. Further, the RNA or cDNA derived therefrom may be hybridized to a solid substrate at a bio-electrical interface whereupon hybridization of a specific RNA, or cDNA derived therefrom, generates an electrical signal which may further be amplified and detected.

The bodily fluid may be either blood plasma or serum. It is preferred, but not required, that blood be processed soon after drawing, and preferably within three hours, as to minimize any nucleic acid degradation in the sample. Blood may be first collected by venipuncture and kept on ice until further processing. Preferably, within 30 minutes to one hour of drawing the blood, serum is separated by centrifugation, for example at 1100 x g for 10 minutes at 4 degrees C. When using plasma, the blood is not permitted to coagulate prior to separation of the cellular and acellular components. Serum or plasma can be frozen, for example at -70 degrees C, after separation from the cellular portion of blood until further assayed, whereupon freezing the specimen can be maintained for extended periods (for example, several years) prior to assaying. When using frozen blood plasma or serum or other bodily fluid, the frozen serum or plasma or bodily fluid is rapidly thawed, for example in a 37 degree C water bath, and RNA is extracted therefrom without delay using methods as described above.

Following the extraction of RNA from a bodily fluid of an animal, a fraction of which contains a mammalian RNA associated with a non-neoplastic disease or pathologic disease or injury, or a fraction of which contains a mammalian RNA derived from cells or tissues of an organ or organ system of said animal, including but not limited to RNA derived from non-proliferating cells and tissues, and/or RNA derived from terminally differentiated cells and tissues, the RNA or cDNA derived therefrom is preferably amplified *in vitro.* Applicable amplification assays include but are not limited to amplifications assays detailed in co-owned U.S. Patent No. 6,329,179 and include but are not limited to reverse transcriptase polymerase chain reaction (RT-PCR), ligase chain reaction, RNA and cDNA signal amplification methods including branched chain signal amplification, amplifiable RNA reporters, Q-beta replication, transcription-based amplification, boomerang DNA amplification, strand displacement activation, cycling probe technology, isothermal. nucleic acid sequence based amplification, other self sustained sequence replication assays, and other nucleic acid amplification assays as known in the art, and/or any variations or combinations thereof, performed in either qualitative or quantitative fashion. For example, the methods can utilize nucleic acid amplification methods as known in the art, such as but not limited to adapting those described by Edmands et al. (1994 PCR Methods Applic. 3: 317-319); Abravaya et al. (1995 Nucleic Acids Res. 23: 675-682); Urdea et al. (1993 AIDS 7 (suppl 2): S11-S14); and/or Kievits et al. (1991 J. Virological Methods 35: 273-286);

In the methods, mammalian RNA may be converted into cDNA using reverse transcriptase prior to *in vitro* amplification using methods known in the art. For example, a sample, such as 10 microL extracted serum RNA is reverse transcribed in a 30 microL volume containing 200 Units of Moloney murine leukemia virus (MMLV) reverse transcriptase (Promega, Madison, WI), a reaction buffer supplied by the manufacturer, 1 mM dNTPs, 0.5 micrograms random hexamers, and 25 Units of RNAsin (Promega, Madison, WI). Reverse transcription is typically performed under an overlaid mineral oil layer to inhibit evaporation and incubated at room temperature for 10 minutes followed by incubation at 37 degrees C for one hour. Alternatively, other methods well known in the art can be used to reverse transcribe the mammalian RNA to cDNA.

Amplification primers or probes may be specific for amplifying the mammalian RNA or cDNA derived therefrom associated with a non-neoplastic disease or pathologic condition, and/or associated with a non-neoplastic and/or terminally differentiated and/or non-proliferative tissue from an organ or organ system. Amplification may be performed by RT-PCR, wherein amplification primers are specific for amplifying the cDNA. It is to be recognized that the design of said primers or probes is based upon the nucleic acid sequence of the RNA or cDNA, as known in the art, using methods as known in the art, and further as detailed in co-owned U.S. Patent No. 6,329,179 B1.

In the methods, following amplification the amplification product of the RNA or cDNA, or the amplified signal product of the RNA or cDNA, may then be detected in either a qualitative or quantitative fashion. In the methods, detecting an amplification product of the mammalian RNA or cDNA derived therefrom, or signal derived therefrom, is accomplished using a detection method such as but not limited to gel electrophoresis; capillary electrophoresis; conventional enzyme-linked immunosorbent assay (ELISA) or modifications thereof, such as amplification using biotinylated or otherwise modified primers; nucleic acid hybridization using specific, detectably-labeled probes, such as fluorescent-, radioisotope-, or chromogenically-labeled probe; laser-induced fluorescence detection; Northern blot analysis; Southern blot analysis; electrochemiluminescence; reverse dot blot detection; and high-performance liquid chromatography, wherein the methods of detection are performed using methods known in the art.

In one example cardiac troponin T mRNA is detected in a bodily fluid, most preferably blood, blood plasma, or serum, or in other bodily fluid. Detection of cardiac troponin T mRNA in a bodily fluid is advantageous for the detection, diagnosis, monitoring, prognosticating, or providing a predictive indicator of non-neoplastic diseases and pathologic conditions of the heart, most preferably myocardial infarction, subclinical myocardial infarction and injury, and/or coronary insufficiency, including that associated with angina and unstable angina. Amplification may be performed by RT-PCR, preferably by the method of Townsend et al. (1995 J. Mol. Cell. Cardiol. 27: 2223-2236), or Messner et al. (2000 Am. J. Clin. Pathol. 114: 544-549) or Ricchiuti and Apple (1999 Clin. Chem. 45: 2129-2135); The method set forth by Messner et al. (2000 Am. J. Clin. Pathol: 114: 544-549) may be used, wherein nested RT-PCR is performed, wherein the preferred oligonucleotide primer sequences used in the first RT-PCR amplification reactions are as follows:

| | |
|---|---|
| Primer 1: | 5' GTTCTGAGGGAGAGCAGA (Sense; SEQ ID No. 1) |
| Primer 2: | 5' AAGTGGTTTCTAGACGAGGA (Antisense; SEQ ID No. 2) |

And wherein the preferred oligonucleotide primer sequences used in the second RT-PCR amplification reactions are as follows:

| | |
|---|---|
| Primer 3: | 5' GACCATGTCTGACATAGAAG (Sense; SEQ ID No. 3) |
| Primer 4: | 5' CCGTCTCGTAGATATTGAAC (Antisense; SEQ ID No. 4) |

In one example, cardiac troponin T mRNA is harvested from serum or plasma, for example from an approximately 1.5 mL aliquot of serum or plasma, and RNA extracted therefrom the Perfect RNA Total RNA Isolation Kit (Five Prime-Three Prime) according to manufacturer's directions. From this extracted RNA preparation, 10 microL are then reverse transcribed to cDNA as described above. Nested RT-PCR for the cardiac troponin T cDNA is performed using the method of Messner et al. (2000 Am. J. Clin. Pathol. 114: 544-549) wherein PCR is performed using *Taq* DNA Polymerase and the Incubation Mix (with 1.5 mmol/L MgCl₂) from Appligene Oncor (Illkirch Cedex, France). Primers 1-4 as described above (SEQ ID Nos. 1-4) are utilized, with Primers 1 and 2 (SEQ ID Nos. 1 and 2) added to the mixture for the first stage of the PCR reaction, and Primers 3 and 4 (SEQ ID Nos. 3 and 4) added to the mixture for the second stage of the PCR reaction, for example using 10 picomoles each of Primer 1 and 2 (SEQ ID Nos. 1 and 2), and 10 picomoles each of Primer 3 and 4 (SEQ ID Nos. 3 and 4). The appropriate mixtures for each stage reaction are amplified in a thermocycler under a temperature profile consisting of 30 cycles of denaturation at 94 degrees C for 30 seconds, annealing at 60 degrees C for 30 seconds, and extension at 72 degrees C for 1 minute. Detection of the amplified product is then achieved, for example, by gel electrophoresis through a 1.5% agarose gel (Molecular Biology Grade Agarose, Gibco BRL), using ethidium bromide staining for visualization and identification of the product fragment, wherein the expected length for the cTnT3 isoform is 733 base pairs, and the expected length for the cTnT4 isoform is 634 base pairs.

The disclosure also illustrates alternative methods of amplification of cardiac troponin T mRNA or cDNA known in the art, including but not limited to the methods of Ricchiuti and Apple (1999 Clin. Chem. 45: 2129-2135), , and of Townsend et al. (1995 J. Mol. Cell. Cardiol 27: 2223-2236),

The disclosure further illustrates the cloning of the amplified product fragments into recombinant DNA replication vectors using standard techniques, for example for the cloning of cTnT mRNA or cDNA amplified products into pGEM-T vectors as described by Townsend et al (1995 J. Mol. Cell. Cardiol. 27: 2223-2236), RNA can be produced from cloned PCR products, and in some instances the RNA expressed thereby, for example by using the Quick Coupled Transciption/Translation kit (Promega, Madison, WI) as directed by the manufacturer.

The disclosure further illustrates restriction enzyme digestion of an amplified product, such as the restriction enzyme digestion of a cTnT mRNA or cDNA amplified product and/or cTnI mRNA or cDNA amplified product, such as using the restriction enzymes *Hinf*I and *Msp*I (New England BioLabs, Beverly, MA), as described by Messner et al. (2000 Clin. Chem. 114: 544-549), It will further be recognized that amplified products can be restriction enzyme digested prior to a second stage of amplification. Amplification methods can also be performed using primers specific for an internal control sequence, such as glyceraldehyde-3-phosphate dehydrogenase or c-abl, using methods as known to the art.

In another example, cardiac troponin I mRNA (cTnI mRNA) is detected in a bodily fluid, most preferably blood, blood plasma, or serum, or in other bodily fluid. Detection of cardiac troponin I mRNA in a bodily fluid is advantageous for the detection, diagnosis, monitoring, prognosticating, or providing a predictive indicator of non-neoplastic diseases and pathologic conditions of the heart, most preferably myocardial infarction, subclinical myocardial infarction or injury, and/or coronary insufficiency, including that associated with angina and unstable angina. In a preferred embodiment, amplification is performed by RT-PCR, preferably by the method of Messner et al. (2000 Am. J. Clin. Pathol. 114: 544-549), or Ricchiuti and Apple (1999 Clin. Chem. 45: 2129-2135), In a preferred embodiment, the method of Messner et al. (2000 Am. J. Clin. Pathol. 114: 544-549) is used, wherein nested RT-PCR is performed, wherein the preferred oligonucleotide primer sequences used in the first RT-PCR amplification reactions are as follows:

| | |
|---|---|
| Primer 1: | 5' AACCTCGCCCTGCACCAG (Sense; SEQ ID No. 5) |
| Primer 2: | 5' CCCGGGACTCCTTATTTCG (Antisense; SEQ ID No. 6) |

And wherein the preferred oligonucleotide primer sequences used in the second RT-PCR amplification reactions are as follows:

| | |
|---|---|
| Primer 3: | 5' CCTCCAACTACCGCGCTTA (Sense; SEQ ID No. 7) |
| Primer 4: | 5' GACTCGGAAGGACGGATGA (Antisense; SEQ ID No. 8) |

In one example cardiac troponin I mRNA is harvested from serum or plasma, for example from an approximately 1.5 mL aliquot of serum or plasma, and RNA extracted therefrom the Perfect RNA Total RNA Isolation Kit (Five Prime-Three Prime) according to the manufacturer's directions. From this extracted RNA preparation, 10 microL are then reverse transcribed to cDNA as described above. Nested RT-PCR for the cardiac troponin I cDNA is performed using the method of Messner et al. (2000 Am. J. Clin. Pathol. 114: 544-549) wherein PCR is performed using *Taq* DNA Polymerase and the Incubation Mix (with 1.5 mmol/L MgCl₂) from Appligene Oncor (Illkirch. Cedex, France). Primers 1-4 as described above (SEQ ID Nos. 5-8) are utilized, with Primers 1 and 2 (SEQ ID Nos. 5 and 6) added to the mixture for the first stage of the PCR reaction, and Primers 3 and 4 (SEQ ID Nos. 7 and 8) added to the mixture for the second stage of the PCR reaction, for example using 10 picomoles each of Primer 1 and 2 (SEQ ID Nos. 5 and 6), and 10 picomoles each of Primer 3 and 4 (SEQ ID Nos. 7 and 8). The appropriate mixtures for each stage reaction are amplified in a thermocycler under a temperature profile consisting of 30 cycles of denaturation at 94 degrees C for 30 seconds, annealing at 60 degrees C for 30 seconds, and extension at 72 degrees C for 1 minute. Detection of the amplified product is then achieved, for example, by gel electrophoresis through a 1.5% agarose gel (Molecular Biology Grade Agarose, Gibco BRL), using ethidium bromide staining for visualization and identification of the product fragment, wherein the expected length for the cTnI amplification product is 581 base pairs.

The disclosure illustrates alternative methods of amplification of cardiac troponin I mRNA or cDNA known in the art, including but not limited to the methods of Ricchiuti and Apple (1999 Clin. Chem. 45: 2129-2135).

In another example beta-myosin heavy chain mRNA is detected in a bodily fluid of an animal, most preferably in blood, blood plasma, or serum or in other bodily fluid. Detection of beta-myosin heavy chain mRNA in a bodily fluid is advantageous for the detection, diagnosis, monitoring, prognosticating, or providing a predictive indicator of non-neoplastic diseases and pathologic conditions of muscle, most advantageously cardiac muscle of the heart. In one example beta-myosin heavy chain mRNA is harvested serum or plasma, for example from an approximately 1.5 mL aliquot of serum or plasma, and RNA extracted therefrom using the Perfect RNA Total RNA Isolation Kit (Five Prime- Three Prime) according to the manufacturer's directions. From this extracted RNA preparation, 10 microL are then reverse transcribed to cDNA as described above. The cDNA is then hybridized to a primer or probe specific to beta-myosin heavy chain cDNA, most preferably an oligonucleotide primer or probe, wherein the primer or probe is specific to the nucleotide sequence of a fragment of beta-myosin heavy chain cDNA. Alternatively, extracted mRNA may be hybridized directly to a probe specific to the nucleotide sequence of a fragment of the mRNA. Hybridized primers or probes may thereby enable either qualitative or quantitative amplification or signal amplification of the mRNA or cDNA derived therefrom, such as beta-myosin heavy chain cDNA, followed by detection of the product, by methods of the art as previously described.

In another example acidic fibroblast growth factor mRNA and/or basic fibroblast growth factor mRNA is detected in a bodily fluid, most preferably blood, blood plasma and serum, or other bodily fluid. Detection of acidic fibroblast growth factor mRNA and/or basic fibroblast growth factor mRNA in a bodily fluid is advantageous for the detection, diagnosis, monitoring, prognosticating, or providing a predictive indicator of non-neoplastic diseases and pathologic conditions of the cardiovascular system, most preferably non-neoplastic diseases and pathologic conditions relating to atherosclerosis and intimal hyperplasia. Amplification may be performed by RT-PCR, preferably by the method of Zhao et al. (1994 Circulation 90: 677-685) but preferably for 45 cycles (full stop).

In one example, acidic fibroblast growth factor mRNA and/or basic, fibroblast growth factor mRNA is harvested from blood, most preferably blood plasma or serum, or other bodily fluid, for example from an approximately 1.5 mL aliquot of serum or plasma, and RNA extracted therefrom using the Perfect RNA Total RNA Isolation Kit (Five Prime- Three Prime) according to the manufacturer's directions. From this extracted RNA preparation, 10 microL are then reverse transcribed to cDNA as described above. RT-PCR for acidic fibroblast growth factor cDNA and/or basic fibroblast growth factor cDNA is performed using the method of Zhao et al. (1994 Circulation 90:677-685) but preferably for 45 cycles, with the amplified product detected as previously described, for example by gel electrophoresis with ethidium bromide staining.

In another example prostate apoptosis response-4 (Par-4) mRNA is detected in a bodily fluid, most preferably cerebrospinal fluid, or blood, blood plasma, serum, or other bodily fluid. Detection of Par-4 mRNA in a bodily fluid is advantageous for the detection, diagnosis, monitoring, prognosticating, or providing a predictive indicator of non-neoplastic diseases and pathologic conditions and injuries of the brain and nervous system, such as stroke, ischemia of the brain, hypoxia of the brain, traumatic brain injury, and neurodegenerative disease. Amplification may be performed by RT-PCR, preferably by the method of Dhillon et al. (2001 Exp. Neurol. 170: 140-148) but preferably for 45 cycles.

In one example, Par-4 mRNA is harvested from cerebrospinal fluid or serum or plasma, for example from an aliquot of cerebrospinal fluid, and RNA extracted therefrom using the Perfect RNA Total RNA Isolation Kit (Five Prime-Three Prime) according to manufacturer's directions. From this extracted RNA preparation, 10 microL are then reverse transcribed to cDNA as described above. RT-PCR for Par-4 cDNA is performed using the method of Dhillon et al. (2001 Exp. Neurol. 170: 140-148) but preferably for 45 cycles. with the amplified product detected as previously described, for example by gel electrophoresis with ethidium bromide staining.

In particularly advantageous methods, a multiplexed panel or sequential analysis or cDNA chip approach is employed to allow the concurrent or sequential analysis of multiple RNA from a bodily fluid specimen. In one aspect of this embodiment, multiple mammalian RNA associated with a particular organ or organ system are thereby detected in a bodily fluid, most preferably blood, blood plasma, serum, or other bodily fluid, as a method for detecting, diagnosing, monitoring, predicting, or prognosticating a non-neoplastic disease or pathologic condition or injury. In a particularly advantageous embodiment cardiac troponin T mRNA and cardiac troponin I mRNA or other myocardial-derived RNA such as beta-myosin heavy chain mRNA are detected in sequential, concurrent, multiplexed, or chip fashion from the same bodily fluid specimen, most preferably blood, blood plasma, serum, or other bodily fluid.

In an embodiment the RNA of interest is compared to RNA from a housekeeper gene or genes similarly extracted from the bodily fluid in either quantitative or qualitative fashion.

In another embodiment, mammalian RNA from a bodily fluid specimen of an animal, most preferably a human, is concurrently or sequentially analyzed in comparison with protein markers or lipoprotein markers or DNA markers from said bodily fluid specimen in qualitative or quantitative fashion; wherein comparative analysis of the presence of mammalian RNA, in said bodily fluid specimen to the presence of the protein or DNA in said bodily specimen facilitates the diagnosis, detection, evaluation, monitoring, prognosticating, or predicting of a non-neoplastic disease or pathologic condition or injury in said animal. For example, mammalian RNA such as but not limited to acidic fibroblast growth factor mRNA and/or basic fibroblast growth factor mRNA and/or platelet-derived growth factor mRNA may be detected in blood and sequentially or concurrently compared with serum lipoproteins and/or serum cholesterol as a method of prognosticating or predicting atherosclerotic disease.

The examples of preferred embodiments provided herein whereby cardiac troponin T mRNA or cardiac troponin I mRNA or beta-myosin heavy chain mRNA or acidic fibroblast growth factor mRNA or basic fibroblast growth factor mRNA or Par-4 mRNA are detected in bodily fluid are provided as examples and not as limitations on the methods of the invention. It is to be understood that the invention generally encompasses detection of extracellular mammalian RNA associated with traumatic orischemic injury of the human heart or brain, wherein the RNA is detected in a bodily fluid taken from said human. It will be understood in the art that other RNA may provide markers of said injury, and it is within the scope and spirit of the invention that these RNA may be extracted as extracellular RNA from blood plasma, serum, or other bodily fluid, the RNA species of interest or cDNA derived therefrom can be amplified or signal amplified using primers or probes specific to the RNA or cDNA of interest, and the amplified product or signal be detected, as is taught by the invention herein.

In a particularly preferred embodiment, the mammalian RNA associated with a non-neoplastic disease or pathologic condition or cDNA derived therefrom is amplified or signal amplified in a quantitative amplification reaction. Quantitative amplification of the mammalian RNA or cDNA is particularly advantageous when said RNA is present at lower levels in a bodily fluid of healthy animals, but present at higher levels in a bodily fluid of animals with a disease or pathologic condition or injury. The method thereby enables statistically-based discrimination between individuals with a disease or pathologic condition and healthy populations or populations without the disease or pathologic condition. The quantitative method further enables comparison between individuals having the disease or condition, wherein higher levels of said RNA in a bodily fluid is indicative of a disease or pathologic condition of greater severity, or of earlier onset. The quantitative method thereby provides a method for monitoring a disease or pathologic condition, or monitoring a response to therapy for a disease or pathologic condition, or for determining a prognosis. The methods of the disclosure thereby illustrate a marker for assessing the adequacy of therapy, or for determining whether additional or more advanced therapy is required. It is particularly advantageous to perform the methods of the disclosure in a serial manner to monitor an animal's disease or condition, and to assess the adequacy of therapy or the need to change therapy. The methods thereby further permit rational, informed treatment options to be used for making therapeutic decisions.

The methods are thereby advantageously used for providing a diagnosis or prognosis of, or as a predictive indicator for a non-neoplastic disease or pathologic condition or injury. The methods are particularly useful for providing a diagnosis or prognosis of, or monitoring of, or for providing a predictive indicator for cardiovascular diseases and conditions. Thus, the methods will be useful in the assessment of individuals having symptoms that might be consequent to a cardiovascular disease or condition. The methods will further be useful in the assessment of individuals having risk factors for a cardiovascular disease or condition. The methods will further be useful for the monitoring or determining prognosis of individuals known to have a cardiovascular disease or condition. The methods will thus be useful either alone or in conjunction with other tests, assays, procedures, or exams that enable the evaluation of cardiovascular diseases and conditions, such as but not limited to stress tests, radiologic scans, echocardiogram, and electrocardiograms. The methods will further be useful to monitor an individual during or following surgery.

The methods are further particularly useful for providing a diagnosis or prognosis of, or as a predictive indicator for a non-neoplastic neurologic disease or neurologic pathologic condition or injury. Thus, the methods will be useful in the assessment of individuals having symptoms that might be consequent to a neurologic disease or condition. The methods will further be useful in the assessment of individuals having risk factors for a neurologic disease or condition. The methods will further be useful for the monitoring or determining prognosis of individuals known to have a neurologic disease or condition. The methods will thus be useful either alone or in conjunction with other tests, assays, procedures, or exams that enable the evaluation of neurologic diseases and conditions, such as but not limited to radiologic exams such as CT scan and MRI scan, electroencephalogram, and lumbar puncture. The methods will further be useful to monitor an individual during or following surgery.

The methods will further be advantageous in the screening of individuals for predisposition to diseases and pathologic conditions, thereby enabling the institution of preventive therapy.

The methods provides for diagnostic kits for the detection, diagnosis, monitoring, prognosticating, or predicting of non-neoplastic disease or pathologic condition or injury, wherein the diagnostic kit provides for the extraction of mammalian RNA from plasma, serum, or other bodily fluid, and/or provides primers or probes used in the detection of the extracted RNA of interest or cDNA derived therefrom.

The methods and preferred uses for the methods of the invention are more fully illustrated in the following Example. This Example illustrates certain aspects of the above-described method and advantageous results. This Example is shown by way of illustration and not by way of limitation.

### EXAMPLE 1

A 52 year-old man presents to his doctor with complaints of recent onset of increasingly frequent episodes of mild chest discomfort. His doctor suspects a possible cardiac etiology, and orders further cardiac evaluation. The man undergoes a "stress test" consisting of an electrocardiogram test during and following controlled treadmill exercise. Peripheral venous blood is drawn from the man one hour and six hours following the stress test to evaluate for the presence of cardiac troponin T mRNA and cardiac troponin I mRNA using the methods of the invention. Five ml of blood plasma is collected for each time period, maintained on ice until separation of plasma from the cellular blood fraction, and then frozen until further testing. Both plasma samples are evaluated in a laboratory at the same time by rapidly thawing the frozen samples, extracting RNA from the plasma using a commercial RNA extraction kit such as the Perfect RNA Total RNA Isolation Kit (Five Prime-Three Prime) according to manufacturer's directions, reverse transcribing the extracted RNA to cDNA as previously described, and amplifying the cDNA with primers specific for cardiac troponin T cDNA and cardiac troponin I cDNA by the methods of the invention, such as by using the method of Messner et al. (2000 Am. J. Clin. Pathol. 114: 544-549), performed in a qualitative fashion. The amplified product is then detected, such as by using gel electrophoresis. Detection of cardiac troponin T mRNA and/or cardiac troponin I mRNA in the peripheral blood would indicate an underlying cardiovascular disease associated with cellular injury during the stress test, and the doctor in this case would thereby make a diagnosis of unstable angina and would thereby institute therapeutic measures.

Five weeks following the treadmill the patient presents to the emergency room with complaints of sustained substernal chest discomfort and shortness of breath. The emergency doctor suspects a possible myocardial infarction. To confirm this, he obtains peripheral venous blood from the patient and evaluate the blood for the presence of cardiac troponin T mRNA and/or cardiac troponin I mRNA in the peripheral blood, using the methods of the invention described. The presence of cardiac troponin T mRNA and cardiac troponin I mRNA is thereby confirmed and a diagnosis of myocardial infarction thereby made, and the man is admitted to the hospital coronary care unit. There, cardiac troponin T mRNA and cardiac troponin I mRNA in blood would be serially quantitatively monitored using the method of the invention as a means of monitoring the progression of the myocardial infarction, the severity of the myocardial tissue injury, and the prognosis for the patient. In addition, plasma beta-myosin heavy chain mRNA is monitored using the method of the invention to further evaluate the severity of the myocardial tissue injury.

## Claims

1. A method for detecting, diagnosing or monitoring, an injury of an organ in a human, the method comprising the step of detecting qualitatively or quantitatively extracellular human RNA in a bodily fluid previously obtained from said human, wherein said human RNA is detected by assaying for said human RNA or cDNA derived therefrom, in a qualitative or a quantitative fashion,
wherein the injury of an organ is traumatic or ischemic injury of the brain or heart.

2. The method of claim 1, wherein traumatic or ischemic injury of the brain or heart is a stroke, myocardial infarction, myocardial ischemia, ischemic brain injury, hypoxia of the brain, or head trauma.

3. The method of any of claims 1 to 2, wherein the bodily fluid is selected from blood, blood plasma, urine, cerebrospinal fluid or serum.

4. A method for detecting extracellular human RNA in blood, blood plasma or serum, or other bodily fluid, all of them, blood, blood plasma, serum, or other bodily fluid, previously obtained from a human, wherein said RNA is associated with an injury of an organ of said human, the method comprising the steps of:
a) extracting RNA from blood, blood plasma, serum, or other bodily fluid from a human, wherein a portion of said extracted RNA comprises an extracellular human RNA associated with an injury of an organ of said human;
b) *in vitro* amplifying or signal amplifying a fraction of the extracted RNA or cDNA derived therefrom in a qualitative or quantitative fashion using primers or probes specific for a human nucleic acid sequence of said extracellular human RNA, or cDNA derived therefrom, to produce an amplified product or signal;
c) detecting the amplified product or signal produced thereby,
wherein the injury of an organ is traumatic or ischemic injury of the brain or heart.

5. The method of claim 4, wherein said extracellular human RNA is derived from heart, or brain of said human.

6. The method according to claim 4 or 5, whereby an injury of an organ is detected, diagnosed or monitored.

7. The method according to claims 4 to 6, wherein said organ is heart or brain of the human.

8. The method according to any of claims 4 to 7, wherein the human RNA translates a protein that has a deleterious effect upon cells or tissues within the human.

9. A method of detecting extracellular human RNA obtained from plasma or serum from a human, said human RNA being associated with an injury of an organ of said human, the method comprising the steps of:
a) extracting RNA from blood plasma or serum from a human, wherein a portion of said RNA comprises an extracellular human RNA associated with an injury of an organ of said human;
b) hybridizing a portion of said extracellular human RNA associated with an injury of an organ of said human, or cDNA derived therefrom, to a primer, probe, or solid substrate, wherein the primer, probe, or solid substrate is specific for human nucleotide sequences of said extracellular human RNA, or cDNA derived therefrom;
c) detecting the hybridized RNA or cDNA,
wherein the injury of an organ is a traumatic or ischemic injury of the brain or heart.

10. The method of claim 4 or 9, wherein said human RNA is cardiac troponin T mRNA, cardiac troponin I mRNA, beta-myosin heavy chain mRNA, acidic fibroblast growth factor mRNA, or Par-4 mRNA.

11. The method of claim 9, wherein said extracellular human RNA is derived from heart or brain.

12. The method of claim 4 or 9, wherein said injury of an organ is one from a group comprising myocardial infarction, myocardial ischemia, congestive heart failure, cardiomyopathy, stroke, ischemic brain injury, hypoxic conditions of the brain, and head trauma.

## Patentansprüche

1. Ein Verfahren zum Nachweisen, Diagnostizieren oder Überwachen einer Verletzung eines Organs in einem Menschen, wobei das Verfahren folgendes umfasst:
den Schritt des Nachweisens von extrazellulärer menschlicher RNA auf qualitativem oder quantitativem Weg in einer Körperflüssigkeit, die zuvor von dem Menschen erhalten wurde, wobei besagte menschliche RNA nachgewiesen wird durch Untersuchen auf besagte menschliche RNA oder cDNA, die daraus abgeleitet ist, in einer qualitativen oder quantitativen Art und Weise,
wobei die Verletzung eines Organs eine traumatische oder ischämische Verletzung des Gehirns oder Herzens ist.

2. Das Verfahren von Anspruch 1, wobei die traumatische oder ischämische Verletzung des Gehirns oder Herzens ein Schlaganfall, ein Myocardialinfarkt, myocardiale Ischämie, ischämische Gehirnverletzung, Hypoxie des Gehirns oder Schädeltrauma ist.

3. Das Verfahren von irgendeinem der Ansprüche 1 oder 2, wobei die Körperflüssigkeit ausgewählt ist aus Blut, Blutplasma, Urin, cerebrospinaler Flüssigkeit oder Serum.

4. Ein Verfahren zum Nachweis von extrazellulärer menschlicher RNA im Blut, Blutplasma oder Serum, oder einer anderen Körperflüssigkeit, wobei alle davon, nämlich Blut, Blutplasma, Serum, oder andere Körperflüssigkeit, zuvor von einem Menschen erhalten wurden, wobei besagte RNA mit einer Verletzung eines Organs assoziiert ist von besagten Menschen, wobei das Verfahren die folgenden Schritte umfasst:
a) Extrahieren von RNA aus Blut, Blutplasma, Serum oder einer anderen Körperflüssigkeit aus einem Menschen, wobei ein Teil von besagter extrahierter RNA eine extrazelluläre menschliche RNA umfasst, die mit einer Verletzung eines Organs von besagten Menschen assoziiert ist;
b) *in vitro* Amplifizieren oder Signal-Amplifizieren eines Teils der extrahierten RNA oder cDNA, die davon abgeleitet ist, in einer qualitativen oder quantitativen Art und Weise unter Verwendung von Primer oder Sonden, die spezifisch für eine menschliche Nukleinsäuresequenz von besagter extrazellulärer menschlicher RNA sind, oder der cDNA, die davon abgeleitet ist, um ein amplifiziertes Produkt oder Signal zu erzeugen;
c) Nachweisen des amplifizierten Produktes oder des Signals, das **dadurch** erzeugt wurde,
wobei die Verletzung eines Organs eine traumatische oder ischämische Verletzung des Gehirns oder des Herzens ist.

5. Das Verfahren von Anspruch 4, wobei besagte extrazelluläre menschliche RNA abgeleitet von Herz oder Gehirn von besagtem Menschen ist.

6. Das Verfahren gemäß Anspruch 4 oder 5, wobei eine Verletzung eines Organs nachgewiesen, diagnostiziert oder überwacht wird.

7. Das Verfahren gemäß den Ansprüchen 4 bis 6, wobei besagtes Organ Herz oder Hirn von besagtem Menschen ist.

8. Das Verfahren gemäß irgendeinem der Ansprüche 4 bis 7, wobei die menschliche RNA ein Protein translatiert, das eine schädliche Wirkung auf Zellen oder Gewebe innerhalb des Menschen ausübt.

9. Ein Verfahren zum Nachweisen von extrazellulärer menschlicher RNA, erhalten aus Plasma oder Serum von einem Menschen, wobei besagte menschliche RNA assoziiert ist mit einer Verletzung eines Organs von besagtem Menschen, und das Verfahren folgende Schritte umfasst:
a) Extrahieren von RNA aus Blutplasma oder Serum eines Menschen, wobei ein Teil von besagter RNA eine extrazelluläre menschliche RNA umfasst, die mit einer Verletzung eines Organs von besagtem Menschen assoziiert ist;
b) Hybridisieren eines Teils von extrazellulärer menschlicher RNA, assoziiert mit einer Verletzung eines Organs von besagtem Menschen oder cDNA abgeleitet davon mit einem Primer, einer Sonde oder einem festen Substrat, wobei der Primer, die Sonde oder das feste Substrat spezifisch für menschliche Nukleotidsequenzen von besagter extrazellulärer menschlicher RNA oder cDNA, die davon abgeleitet ist, sind;
c) Nachweis der hybridisierten RNA oder cDNA;
wobei die Verletzung eines Organs eine traumatische oder ischämische Verletzung des Gehirns oder des Herzens ist.

10. Das Verfahren von Anspruch 4 oder 9, wobei besagte menschliche RNA aus dem Herzen stammende Troponin T mRNA ist, aus dem Herzen stammende Troponin I mRNA, mRNA der schweren Kette von Beta-Myosin, mRNA des aciden fibroblasten Wachstumsfaktors oder Par-4 mRNA.

11. Das Verfahren gemäß Anspruch 9, wobei besagte extrazelluläre menschliche RNA abgeleitet ist von Herz oder Gehirn.

12. Das Verfahren von Anspruch 4 oder 9, wobei besagte Verletzung eines Organs eine ist aus einer Gruppe, welche folgende umfasst: Myokard-Infarkt, Myokard-Ischämie, kongestive Herzinsuffizienz, Cardiomyopathie, Schlaganfall, ischämische Gehirn-Verletzung, hypoxische Zustände des Gehirns und Schädeltrauma.

## Revendications

1. Procédé pour détecter, diagnostiquer ou surveiller une lésion d'un organe chez un humain, le procédé comprenant l'étape consistant à détecter qualitativement ou quantitativement de l'ARN humain extracellulaire dans un fluide corporel préalablement obtenu auprès dudit humain, dans lequel ledit ARN humain est détecté par dosage dudit ARN humain ou d'ADNc en dérivant, d'une façon qualitative ou quantitative, dans lequel la lésion d'un organe étant est une lésion traumatique ou ischémique du cerveau ou du coeur.

2. Procédé selon la revendication 1, dans lequel la lésion traumatique ou ischémique du cerveau ou du coeur est un accident cérébrovasculaire, un infarctus du myocarde, une ischémie myocardique, une lésion cérébrale ischémique, une hypoxie du cerveau, ou un traumatisme crânien.

3. Procédé selon l'une quelconque des revendications 1 et 2, dans lequel le fluide corporel est choisi parmi le sang, le plasma sanguin, l'urine, le liquide céphalo-rachidien et le sérum.

4. Procédé pour détecter un ARN humain extracellulaire dans du sang, du plasma sanguin ou du sérum, ou un autre fluide corporel, tous parmi le sang, le plasma sanguin, le sérum et l'autre fluide corporel ayant été obtenus au préalable auprès d'un humain, dans lequel ledit ARN est associé à une lésion d'un organe dudit humain, lequel procédé comprend les étapes consistant à :
a) extraire de l'ARN du sang, du plasma sanguin, du sérum ou d'un autre fluide corporel d'un humain, une partie dudit ARN extrait comprenant un ARN humain extracellulaire associé à une lésion d'un organe dudit humain ;
b) amplifier in vitro ou amplifier le signal d'une fraction de l'ARN extrait ou d'ADNc en dérivant, d'une façon qualitative ou quantitative au moyen d'amorces ou de sondes spécifiques d'une séquence d'acide nucléique humain dudit ARN humain extracellulaire, ou d'ADNc en dérivant, pour produire un produit ou signal amplifié ;
c) détecter le produit ou signal amplifié ainsi produit,
dans lequel la lésion d'un organe est une lésion traumatique ou ischémique du cerveau ou du coeur.

5. Procédé selon la revendication 4, dans lequel ledit ARN humain extracellulaire est issu du coeur ou du cerveau dudit humain.

6. Procédé selon la revendication 4 ou 5, grâce auquel une lésion d'un organe est détectée, diagnostiquée ou surveillée.

7. Procédé selon les revendications 4 à 6, dans lequel ledit organe est le coeur ou le cerveau de l'humain.

8. Procédé selon l'une quelconque des revendications 4 à 7, dans lequel l'ARN humain traduit une protéine qui a un effet délétère sur des cellules ou tissus de l'humain.

9. Procédé pour détecter un ARN humain extracellulaire issu de plasma ou de sérum d'un humain, ledit ARN humain étant associé à une lésion d'un organe dudit humain, lequel procédé comprend les étapes consistant à :
a) extraire de l'ARN du plasma ou sérum sanguin d'un humain, une partie dudit ARN comprenant un ARN humain extracellulaire associé à une lésion d'un organe dudit humain ;
b) hybrider une partie dudit ARN humain extracellulaire associé à une lésion d'un organe dudit humain, ou d'ADNc en dérivant, à une amorce, une sonde ou un substrat solide, l'amorce, la sonde ou le substrat solide étant spécifique de séquences de nucléotides humaines dudit ARN humain extracellulaire, ou d'ADNc en dérivant ;
c) détecter l'ARN ou ADNc hybridé ;
dans lequel la lésion d'un organe est une lésion traumatique ou ischémique du cerveau ou du coeur.

10. Procédé selon la revendication 4 ou 9, dans lequel ledit ARN humain est l'ARNm de troponine T cardiaque, l'ARNm de troponine I cardiaque, l'ARNm de chaîne lourde de bêta-myosine, l'ARNm de facteur de croissance des fibroblastes acide, ou l'ARNm de Par-4.

11. Procédé selon la revendication 9, dans lequel ledit ARN humain extracellulaire est issu du coeur ou du cerveau.

12. Procédé selon la revendication 4 ou 9, dans lequel ladite lésion d'un organe est l'une de l'ensemble comprenant constitué par un infarctus du myocarde, une ischémie myocardique, une insuffisance cardiaque congestive, une cardiomyopathie, un accident cérébrovasculaire, une lésion cérébrale ischémique, un état hypoxique du cerveau, et un traumatisme crânien.
